# EUROPEAN PATENT APPLICATION

(11) **EP 3 604 507 A1**
(43) Date of publication of application: **05.02.2020**
(21) Application number: 18186426.5
(22) Date of filing: 30.07.2018
(51) Int. Cl.: C12N 9/10, C12P 13/00, C12P 7/26

(54) **AN OMEGA-TRANSAMINASE ENZYME**

(71) Applicant: University College Cork-National University of Ireland, Cork, Cork (IE)
(72) Inventor: The designation of the inventor has not yet been filed
(74) Representative: Purdylucey Intellectual Property

(57) **Abstract**

An isolated ω-transaminase enzyme comprising an amino acid sequence of SEQUENCE ID NO: 1, or encoded by a nucleic acid sequence of SEQUENCE ID NO: 2, is provided and is derived from the marine sponge Pseudovibrio sp. Also provided are functional variants of the ω-transaminase enzyme that are isolated, have at least 70% sequence identity with SEQUENC ID NO: 1, and are capable of enantioselectively resolving a racemic amine by transformation to a ketone by oxidative deamination, or enantioselectively resolving a racemic ketone by transformation to an amine by reductive amination, in which the amine and ketone typically contain a stereocentre removed from the reacting site. In one embodiment, the functional variant is capable of enantioselectively transforming a (4S)-tetralone intermediate in the synthesis of sertraline, in which the functional variant displays remote stereospecificity for the tetralone intermediate.

## Description

### Field of the Invention

The present invention relates to an ω-transaminase enzyme exhibiting remote stereoselectivity in the synthesis of amines and ketones of pharmaceutical relevance

### Background to the Invention

Chiral amines and functional groups derived from amines are found in many pharmaceuticals and fine chemicals.¹ In many cases these stereogenic centres are embedded in complex structures and the API often contains at least one other stereocentre which must be enantiopure. In many cases, "setting" the stereochemistry in the second stereocentre is the greater synthetic challenge. Despite the numerous advancements in organic synthesis over the last number of decades, classical resolution remains a reliable and prominent technique in pharmaceutical manufacture.2

Biocatalysis has emerged as an important weapon in the arsenal of the asymmetric chemist.³ The integration of biocatalytic steps in syntheses can have positive implications for the environmental impact of a process but it can also alter synthetic routes, since exploiting the magnificent chemo, regio- as well as stereoselectivity of biocatalysts can open the door to alternative, shorter syntheses.⁴ Due to their prevalence in pharmaceuticals and fine chemicals, chiral amine subunits are high value synthetic targets and biocatalysis represents a highly enantioselective and sustainable route to them. Indeed many elegant biocatalytic routes to chiral amines have been reported utilising a range of enzymatic families.⁵ Hydrolases have been comprehensively investigated as biocatalysts and have found application in (dynamic) kinetic resolutions of chiral amines,⁶ including in the synthesis of norsertraline **2**.⁷ Monoamine oxidases have been used extensively in the deracemization of racemic amines.⁸ A number of biocatalysts have been employed in the reductive amination of imine precursors, often using superstoichiometric amounts of the amine donor.⁹ Another very recently discovered enzyme, a reductive aminase, can enantioselectively transfer primary and secondary amines to a carbonyl moiety, giving rise to a range of secondary and tertiary amine products, in some cases with 1 molar equivalent of the amine donor.¹⁰

Transaminases have also received much attention in this context.¹¹ *ω*-Transaminases (*ω*TAs, E.C. 2.6.1.X) are pyridoxal-5'-phosphate (PLP)-dependent enzymes that catalyse the reversible transfer of an amine group from a donor molecule (*e*.*g*. alanine) to an acceptor molecule containing a carbonyl group. Unlike the *α*-TAs, they do not require a carboxyl moiety adjacent to the reacting site. If the carbonyl compound is a prochiral ketone the product is a chiral primary amine. Since the amine transfer is reversible, TAs can be used in kinetic resolutions, whereby the substrate is a racemic amine and one enantiomer selectively reacts (an oxidative deamination), or an asymmetric reaction *via* a reductive amination. The asymmetric reaction is more synthetically useful since a theoretical yield of 100% is possible, however oxidative deamination is thermodynamically favoured. PLP enzymes are classified according to their fold class; in general, fold type I TAs are (S)-selective enzymes utilising L-alanine as the amine source while fold type IV are (R)-selective and require D-alanine.^{11a} Structurally, TAs are dimeric proteins which have a large and small binding pocket within their active site. Almost without exception, the small pocket can accommodate only a methyl group or a quasi-methyl group such as a - CH₂- within the framework of a cyclohexyl or cyclopentyl moiety. As such, a major limitation of these enzymes is their limited substrate scope.¹² However, their synthetic power has ensured that they have become valuable tools in the synthesis of stereogenically pure primary amines.¹³ Owing to the limited structure of compounds which are accepted by wild type transaminases, the successful utilisation of a transaminase in a process has relied upon finding a mutant enzyme with the desired substrate profile *via* directed evolution and/or (semi-) rational design.¹⁴ Transaminases have been used in the synthesis of pharmaceutical intermediates and APIs, notably in the synthesis of Januvia® (sitagliptin phosphate).¹⁵ Due to the ingenuity and importance of this work, workers at Merck and Codexis were awarded the 2010 Presidential Green Chemistry Award. In the case of sitagliptin, an (*R*)-selective fold type IV TA was used and 11 rounds of directed evolution, resulting in 27 mutations (8% of the entire sequence), were needed to reach the optimal protein with all of the required properties. Recently, another study described how bulky substrate acceptance could be engineered with just 4 mutations in a Fold I class TA.¹⁶ Despite these and other successes, the search for enzymes with novel functionality and/or broad substrate scope is paramount to the continued development of the field, not least because these new enzymes can act as templates for further evolution of the biocatalyst. Heretofore the most common method used for enzyme discovery has been to investigate fully sequenced and annotated genomes of laboratory cultivable strains. This approach seriously limits the potential "catch", since only 0.1-5% of bacteria cultivable in the laboratory, meaning that much of the existing diversity of bacteria are inaccessible.¹⁷ Metagenome mining has recently emerged as an alternative which casts the net much wider and taps into previously unexplored genome niches, with the premise that primary sequence variation could translate into enzymatic activity or substrate specificity beyond what previously described biocatalysts are capable of.¹⁸ We have elucidated our own experiences and assessment of the enormous potential of the marine world to produce exciting novel biocatalysts.¹⁹

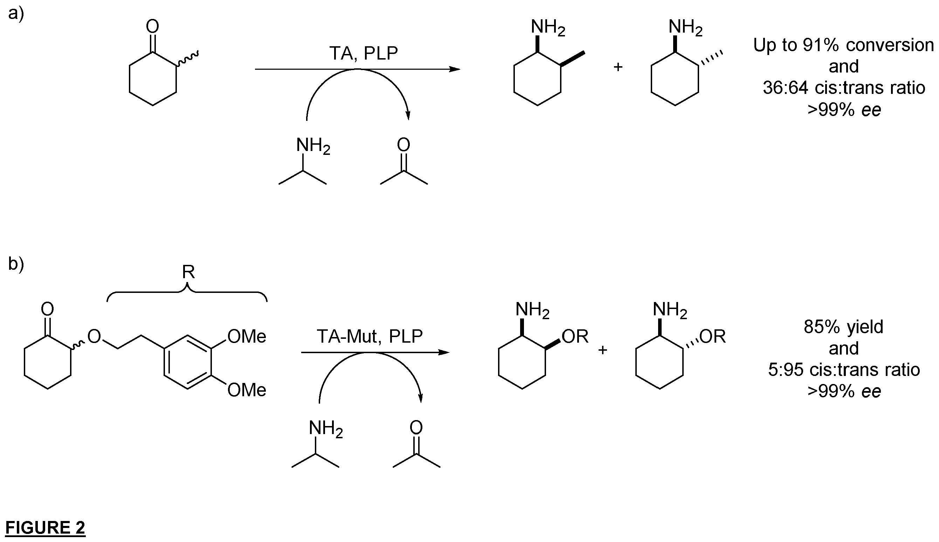

Hydrolases can be used to resolve remote stereocentres,²⁰ and have been particularly important biocatalysts in the context of resolution of APIs and pharmaceutical intermediates.²¹ In some cases more than one stereocentre can be resolved.²² Contrastingly, transaminase-mediated resolutions of compounds bearing even a second stereocentre are very rare. To the best of our knowledge there has been only two such reports and both of these studies were conducted on compounds with a stereogenic centre at the *α*-carbon, taking advantage of the enol equilibrium in a dynamic process (Figure 2). Kroutil and co-workers tested 3 transminases for activity against 2-methylcyclohexanone.²³ In this study, transaminase from *Chromobacterium violaceum* (CV-TA) showed a strong preference for one enantiomer of the ketone starting material. Limanto *et. al.* used a cyclohexanone derivative with a bulky ether substituent, again at the 2-position of the subatrate.²⁴ The amine product was of interest as it is an intermediate in the synthesis of Vernakalant, a drug in development for atrial fibrillation. Interestingly, the original transaminase variant used showed a preference for the wrong ketone enantiomer and 3 rounds of directed evolution (with *in-silico* design) were conducted to find an enzyme with the desired stereopreference. Apart from these two examples, transaminases have been used to set the stereochemistry at the reacting site (i.e. the primary amine) only.

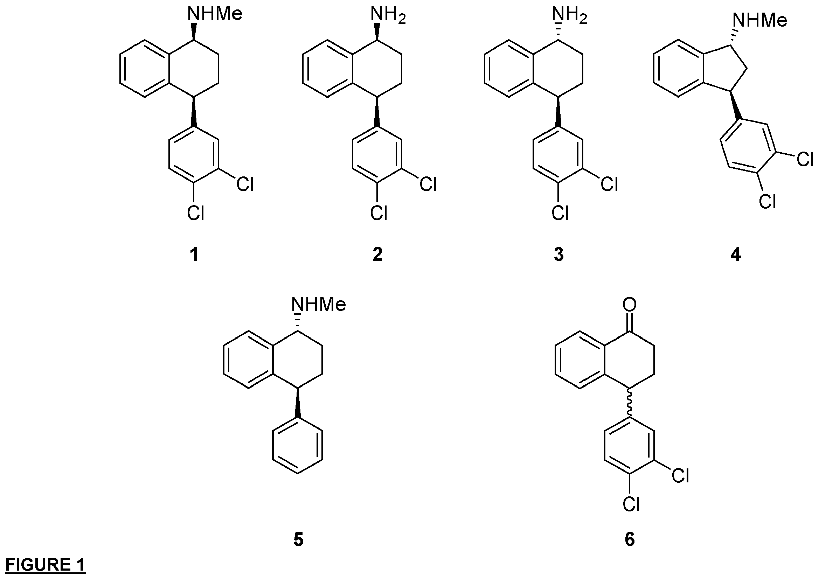

Referring to Figure 1, Sertraline **1** is a Selective Serotonin Reuptake Inhibitor (SSRI) which is produced by Pfizer and it is one of the most prescribed antidepressants in the world.²⁵ Along with a (1*S*)-secondary amine, sertraline contains a stereogenic centre at the 4-position of the aminotetralin core. The aminotetralin bicycle is a privileged subunit in drug discovery, also being present in dasotraline (**3**) and the deschloro (1R)-epimer of sertraline, tametraline (**5**). Dasotraline is the (1*R*)- primary amine equivalent of sertraline and is also a compound of therapeutic interest.²⁶ Desmethylsertraline (norsertraline) **2** is the active metabolite of sertraline and the API can be synthesised from this primary amine precursor. Indatraline **4** is a nonselective monoamine reuptake inhibitor.²⁷

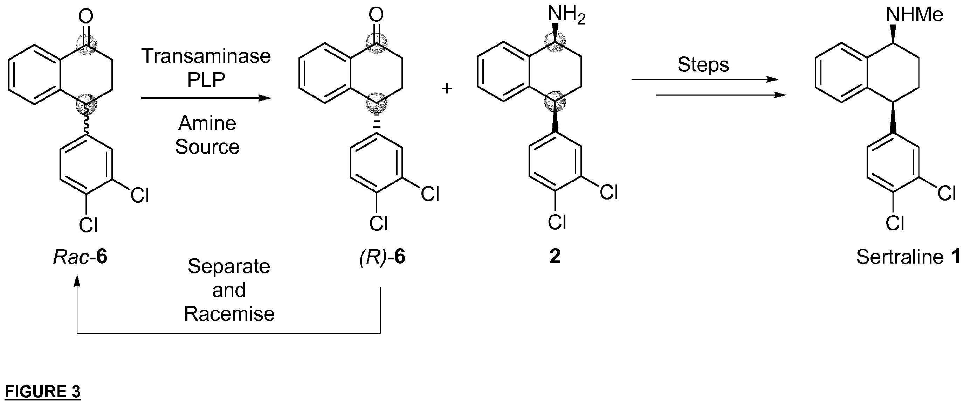

The key intermediate in the synthesis of sertraline itself is tetralone **6** (Figure 3). **6** can be accessed *via* the reaction of 1-napthol and 1,2-dichlorobenzene in the presence of a strong Lewis acid.²⁸ Reductive amination with methylamine yields the product **1** in a 95:5 *cis:trans* ratio.²⁹ Due to the importance of the API, many syntheses have been reported.^{28,30} Despite these efforts, an environmentally friendly, operationally simple method of acquiring (*S*)-**6** remains an important synthetic goal.

It is an object of the invention to overcome at least one of the above-referenced problems and provide an ω-transaminase enzyme(s) exhibiting remote stereoselectivity in the synthesis of amines and ketones of pharmaceutical relevance.

### Summary of the Invention

The present invention addresses the need for an enzyme capable of exhibiting remote stereoselectivity in the synthesis of amines and ketones of pharmaceutical relevance. This enzyme, referred to herein as TA3, is an ω-transaminase derived from a marine sponge *Pseudovibrio* sp. isolate. The sequence of the enzyme (amino acid and nucleic acid) has been identified, and the gene has been cloned into an expression system and expressed as a recombinant protein, which has been employed to enantioselectively transform a model challenging substrate such as the key (4S)-tetralone intermediate in the synthesis of sertraline.

According to a first aspect of the present invention, there is provided an isolated primary ω-transaminase enzyme characterised by comprising an amino acid sequence of SEQUENCE ID NO: 1 (TA3), and functional variants of TA3 having at least 70%, 80%, 90%, or 95% sequence identity with SEQUENCE ID NO: 1 (hereafter "ω-transaminase of the invention").

In one embodiment, the ω-transaminase of the invention is capable of enantioselectively resolving a racemic amine by transformation to a ketone (oxidative deamination), or enantioselectively resolving a racemic ketone by transformation to an amine (reductive amination), in which the amine and ketone typically contain a stereocentre removed from the reacting site. In one embodiment, the ω-transaminase of the invention is capable of enantioselectively transforming the key (4S)-tetralone intermediate in the synthesis of sertraline. In one embodiment, it displays remote stereospecificity for the tetralone intermediate.

In one embodiment, the ω-transaminase of the invention is derived from a bacterium, typically a *Pseudovibrio* bacterium, and in one embodiment a *Pseudovibrio* bacterium of marine origin.

In one embodiment, the ω-transaminase of the invention is isolated from a marine sponge *Pseudovibrium sp* isolate. An example is the ω-transaminase of SEQUENCE ID NO: 1, or encoded by the nucleic acid sequence of SEQUENCE ID NO: 1.

In one embodiment, the ω-transaminase of the invention is a recombinant protein. The invention also provides fusion proteins, or conjugates, comprising an ω-transaminase of the invention.

In one embodiment, the invention provides a nucleic acid encoding an ω-transaminase of the invention. In one embodiment, nucleic acid is a DNA molecule. In one embodiment, the nucleic acid is a cDNA molecule.

In another aspect, the invention provides an expression vector comprising DNA encoding an ω-transaminase of the invention, in which the vector is configured for heterologous expression of the ω-transaminase of the invention in a host cell (hereafter "expression vector of the invention").

In another aspect, the invention provides a host cell, especially a bacterium, yeast, fungus or mammalian producer cell, engineered to heterologously express an ω-transaminase of the invention (hereafter "transformed cell of the invention"). In one embodiment, the transformed host cell comprises an expression vector on the invention.

In another aspect, the invention provides a method of enantioselectively resolving a racemic amine by transformation to a ketone (oxidative deamination), or enantioselectively resolving a racemic ketone by transformation to an amine (reductive amination), in which the amine and ketone typically contain a stereocentre removed from the reacting site, the method comprising a step of reacting the racemic amine or ketone substrate with an ω-transaminase of the invention.

In one embodiment, the ketone substrate is selected from a tetralone and indanone. In one embodiment, the amine substrate is selected from an aminotetralin and an aminoindane. The ketones and amines may have the structures shown below in Formula I and II, and may contain one or additional substitutions:

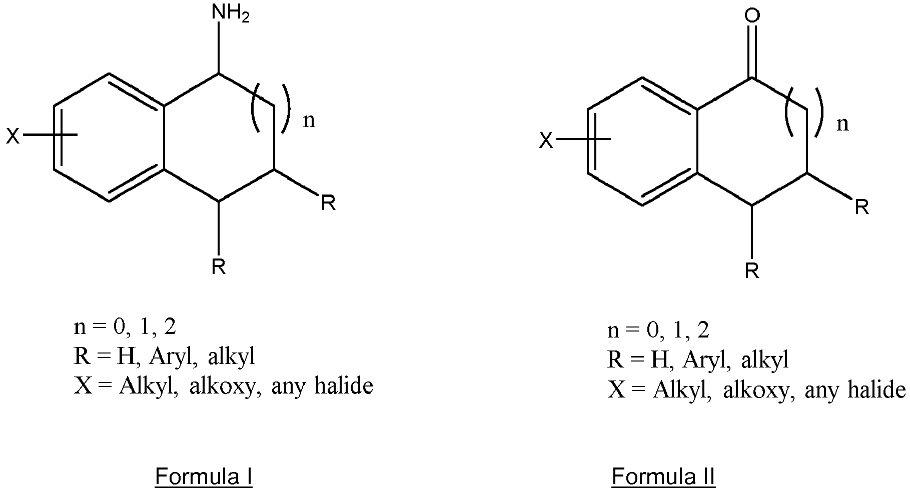

In another aspect, the invention provides a method of enantioselectively reducing 4-(3,4-dichlorophenyl)-3,4-dihydro-1(2H)-naphthalenone (racemic tetralone) to selectively yield enantiopure (3,4-dichlorophenyl)-3,4-dihydro-1(2H)-naphthalamine (4R-tetralone) comprising a step of reacting the racemic tetralone with an ω-transaminase of the invention.

In another aspect, the invention provides a method of producing cis-(1S)(4S)-N-methyl-4-(3,4-dichlorophenyl)-1,2,3,4-tetrahydro-1-naphthaleneamine (sertraline) comprising a step of enantioselectively reducing racemic tetralone to selectively yield 4R-tetralone according to a method of the invention, and chemically converting 4R-tetralone to sertraline.

Thus, the invention relates to the use of the biocatalyst TA3 and functional variants of TA3 to provide access to a range of enantioenriched ketones (including tetralones and indanones) and amines (including aminotetralins and aminoindanes) each of which contains a stereocentre removed from the reacting site, with the tetralone **6**, an intermediate in the synthesis of Sertraline **1**, as an illustrative example. Synthetic use of the biocatalyst for both the reductive amination and the oxidative deamination can be envisaged depending on the desired synthetic outcome and the configuration required

Other aspects and preferred embodiments of the invention are defined and described in the other claims set out below.

### Brief Description of the Figures

**Figure 1****:** Structures of compounds of interest bearing the aminotetralin/aminoindan core.
**Figure 2****:** Previous transaminase work on amines bearing a second stereocentre.
**Figure 3****:** Potential resolution of the remote stereocentre in the tetralone intermediate in the synthesis of sertraline.

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "ω-transaminase of the invention" refers to the isolated enzyme TA3 (SEQUENCE ID NO: 1) and functional variants of the enzyme capable of enantioselectively resolving a racemic amine by transformation to a ketone (oxidative deamination), or enantioselectively resolving a racemic ketone by transformation to an amine (reductive amination), in which the amine and ketone typically contain a stereocentre removed from the reacting site. In one embodiment, the ω-transaminase of the invention is an isolated protein capable of enantioselectively transforming the key model substrate (4S)-tetralone intermediate in the synthesis of sertraline, and which displays remote stereospecificity for the tetralone intermediate. The term includes functional fusion proteins or conjugates comprising the isolated protein. The enzyme may be obtained from a *Pseudovibrio* bacterium, for example a *Pseudovibrio* bacterium obtained from a marine source, for example a marine sponge or other microorganisms form different environments. One example of an ω-transaminase of the invention was isolated from a marine sponge *Pseudovibrio sp* isolate, and is referred to herein as TA3. The amino acid and nucleic acid sequence of TA3 is provided below:
SEQUENCE ID NO: 1 
MDYIANLPPTHVLQEKDAAHHLHPFTDTKSLNAKGTRVITRADGVYLWDSEGNKILDGMA
GLWCVNVGYGRQEIIDAVYRQMQQLPYYNTFFQSSHPPAIGLAERISSLAPDHLDHVFFA
GSGSEANDTVVRMVRHYWASEGKPTKKTIISRHNAYHGSTMAGASLGGMSAMHAQGGL
PIPDITHINQPYWYGEGGDMDPAAFGLMRARELEAEIDRLGEDNVAAFIGEPIQGAGGVIIP
 
PETYWPEIQRICRERNILLIADEVICGFGRTGNWFGSQTFNFKPDLMPIAKGLSSGYLPI
GAVVVSEKVAKGFIEHGGEFYHGFTYSAHPAACAAALANLDIIENERLPEKVANDTGPYL
AEKWKTLGEHPLVGEARICGLVGALELSPDKARRARFEAEKGTVGTICRDHCFESGLVMR
HVGDSMIISPPLVISRSEVDELIQKAHRALDLTAADVTAQSIK
SEQUENCE ID NO: 2

atggactatatcgctaatcttccgcccactcatgttctgcaagaaaaggatgccgcccac
catttgcatccgtttacagataccaagtccctcaacgccaaagggacgcgtgttattacg
cgggctgatggtgtctatctttgggattctgagggcaacaagattctggatggtatggcc
gggctttggtgcgtgaacgtggggtatggccgtcaagagattatcgatgcggtctaccgg
caaatgcaacaactgccgtattacaatacgtttttcagagttctcacccgcctgcaatt
ggtcttgctgaaaggatctccagtctggcaccagatcatctggaccatgtgttctttgca
ggctcgggctctgaagccaatgacaccgtggtgcggatggtgcgccattactgggctagt
gaaggtaagccgaccaagaagacaatcatttcccgtcataatgcttatcacggctccact
atggctggtgcgagcttgggtggtatgagtgcgatgcatgcacagggcggtctacctatt
cctgacattacccatatcaaccagccttattggtatggtgaaggcggagatatggacccct
gccgcgtttggactgatgcgtgcacgtgagctggaagctgagattgaccggcttggagaa
gacaatgtagcggcctttatcggggagcctattcagggagcgggtggtgtgatcattccg
ccagaaacatattggcccgagattcagcgtatctgccgtgaacgcaatattcttctgatt
gctgatgaagtgatttgcgggtttgggcgtacgggtaactggtttgggtcacagactttc
aacttcaaaccggatctgatgccgattgccaaggggctttcctcaggttatctgccgatt
ggtgcggttgtggtgagtgagaaggttgccaagggctttattgagcacggcggtgaattt
tatcatggtttcacttactcggcacatccggcagcttgtgccgcagcacttgcgaacctt
gatattattgagaatgagcgactgccggaaaaagtggcgaatgatacagggccgtatcta
gctgagaaatggaagactcttggcgagcatccgttggttggtgaggcgcgtatctgcggg
ctggttggtgctttggaactttcgcctgataaggcacgccgtgctcgttttgaagctgaa
aaaggcactgttggaacgatctgccgcgatcattgctttgagagcggtttggtgatgcgc
catgttggcgacagcatgatcatctcaccgccacttgtgatctcgcgcagtgaggtggat
gagttgattcagaaagcgcatcgtgcgttggatctgacggctgctgatgttactgcccaa
agcatcaaataa

When necessary, any of the ω-transaminase proteins of the invention can be chemically modified, for example to increase their stability, substrate specificity, or activity. A chemically modified protein or protein analog includes any functional chemical equivalent of the protein Thus, the term ω-transaminase of the invention also refers to any functional amino acid derivative of the protein of SEQUENCE ID NO: 1 as described herein. A protein analog can be produced by procedures that include, but are not limited to, modifications to side chains, incorporation of unnatural amino acids and/or their derivatives during peptide synthesis and the use of cross-linkers and other methods that impose conformational constraint on the peptides or their analogs. Examples of side chain modifications include modification of amino groups, such as by reductive alkylation by reaction with an aldehyde followed by reduction with NaBH4; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH4. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids.

The term "ω-transaminase of the invention" also includes functional variants of the protein of SEQUENCE ID NO: 1, for example variant proteins in which one or more amino acids (for example 1-20, 1-15, 1-10, 9, 8, 7, 6, 5, 4, 3, 2 or 1) are added, deleted or substituted (for example conservative amino acid changes). It is a routine matter for a person skilled in the art to modify the TA3 enzyme by, for example, site directed mutagenesis, or gene editing technology (i.e. CRISPR-CAS9) and test for functionality (Verma et al, Biochim Biophys Acta 2014 Jul: 1884(7):1219-30, Hult et al, Trends in Biotechnology, Vol. 25, Issue 5, May 2007, 231-238). In one embodiment, the modification(s) are made to residues, and made to domains of the protein, which are not catalytically important. In one embodiment, the variant protein has at least 55%, 60%, 70%, 80%, 90%, 91%, 92%, 93%, 94%, 95%, 96%, 97%, 98%, or 99% sequence identity with SEQUENCE ID NO: 1. Calculations of "homology" or "sequence identity" or "similarity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "homology" is equivalent to amino acid or nucleic acid "identity"). The percent identity between the two sequences is a function of the number of identical positions shared by the sequences, taking into account the number of gaps, and the length of each gap, which need to be introduced for optimal alignment of the two sequences.

Amino acid and nucleotide sequence alignments and homology, similarity or identity, as defined herein are preferably prepared and determined using the algorithm BLAST 2 Sequences, using default parameters (Tatusova, T. A. et al, FEMS Microbiol Lett, 174: 187-188 (1999)). Alternatively, the BLAST algorithm (version 2.0) is employed for sequence alignment, with parameters set to default values. BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul, 1990, Proc. Natl. Acad. Sci. USA 87(6):2264-8.

As used herein, the term "isolated" as applied to an ω-transaminase of the invention means a protein or peptide which is isolated from its natural environment, or produced by means of a technical process (i.e. chemical synthesis or recombinant DNA technology).

As used herein, the term "functional" as applied to an ω-transaminase of the invention should be understood to mean that the enzyme is capable of enantioselectively resolving a racemic amine by transformation to a ketone (oxidative deamination), or enantioselectively resolving a racemic ketone by transformation to an amine (reductive amination), in which the amine and ketone typically contain a stereocentre removed from the reacting site. In one embodiment, the variant of TA3 is capable of enantioselectively reducing 4-(3,4-dichlorophenyl)-3,4-dihydro-1(2H)-naphthalenone (racemic tetralone) to selectively yield enantiopure (4S)-((3,4-dichlorophenyl)-3,4-dihydro-1(2H)-naphthalamine (4R-tetralone)enone (4S-tetralone). In one embodiment, the ketone substrate is selected from a tetralone and indanone. In one embodiment, the amine substrate is selected from an aminotetralin and an aminoindane. The ketones and amines may have the structures shown in Formula I and II above:

As used herein, the term "selectively yield" as applied to enantioselective transformation of a racemic ketone or amine substrate should be understood to mean transformation to an enantiopure or enantioenriched product.

As used herein, the term "amine" generally refers to a cyclic amine generally having a stereocentre remote from the ω-transaminase reaction site, for example an aminotetralin or an aminoindane. In one embodiment, the cyclic amine has a structure according to Formula I. As used herein, the term "ketone" generally refers to a cyclic ketone generally having a stereocentre remote from the ω-transaminase reaction site, for example a tetralone or an indanone. In one embodiment, the cyclic ketone has a structure according to Formula II.

"Alkyl" refers to a group containing from 1 to 8 carbon atoms and may be straight chained or branched. An alkyl group is an optionally substituted straight, branched or cyclic saturated hydrocarbon group. When substituted, alkyl groups may be substituted with up to four substituent groups, at any available point of attachment. When the alkyl group is said to be substituted with an alkyl group, this is used interchangeably with "branched alkyl group". Exemplary unsubstituted such groups include methyl, ethyl, propyl, isopropyl, a-butyl, isobutyl, pentyl, hexyl, isohexyl, 4, 4-dimethylpentyl, octyl, 2,2,4-trimethylpentyl, nonyl, decyl, undecyl, dodecyl, and the like. Exemplary substituents may include but are not limited to one or more of the following groups: halo (such as F, Cl, Br, I), Haloalkyl (such as CC13 or CF3), alkoxy, alkylthio, hydroxyl, carboxy (-COOH), alkyloxycarbonyl (-C(O)R), alkylcarbonyloxy (-OCOR), amino (-NH2), carbamoyl (-NHCOOR-or-OCONHR), urea (-NHCONHR-) or thiol (-SH). Alkyl groups as defined may also comprise one or more carbon double bonds or one or more carbon to carbon triple bonds.

The term "halo", as used herein, unless otherwise indicated, includes chloro, fluoro, bromo and iodo.

The term "alkoxy", as used herein, includes O-alkyl groups wherein "alkyl" is defined as above.

The term "aralkyl", as used herein, includes aryl groups, wherein "aryl" is defined as below, terminating in an alkyl group, as defined above, which is the point of attachment.

The term "aryl", as used herein, means mononuclear aromatic hydrocarbon groups such as phenyl, which can be unsubstituted or substituted in one or more positions, and polynuclear aryl groups such as naphthyl, anthryl, phenanthryl, and so forth, which can be unsubstituted or substituted with one or more groups.

The term "one or more substituents", as used herein, includes from one to the maximum number of substituents possible based on the number of available bonding sites. Unless defined otherwise, all technical and scientific terms used herein have the same meaning as commonly understood by one of ordinary skill in the art (e.g., in cell culture, molecular genetics, gene editing, nucleic acid chemistry, hybridization techniques and biochemistry). Standard techniques are used for molecular, genetic and biochemical methods (see generally, Sambrook et al, Molecular Cloning: A Laboratory Manual, 2d ed. (1989) Cold Spring Harbor Laboratory Press, Cold Spring Harbor, N.Y. and Ausubel et al, Short Protocols in Molecular Biology (1999) 4th Ed, John Wiley & Sons, Inc. which are incorporated herein by reference) and chemical methods.

As used herein, the term "*Pseudovibrio bacterium*" refers to a bacterium of the Pseudovibrium genus, including the following species: *Pseudovibrio denitrificans*, *Pseudovibrio ascidiaceicola, Pseudovibrio japonicus, Pseudovibrio axinellae, Pseudovibriohongkongensis*, and *Pseudovibrio stylochi*. As used herein, the term "marine *Pseudovibrio*" or "*Pseudovibrio* of marine origin" refers to a culturable Pseudovibrio bacterium obtained from a marine invertebrate such as a coral, sponge, sea squirt, algae o flatworm or marine sediment. Preferably, the ω-transaminase of the invention is isolated from a marine sponge *Pseudovibrio* bacterium.

As used herein, the term "expression vector of the invention" may be any suitable vector, including chromosomal, non-chromosomal, and synthetic nucleic acid vectors (a nucleic acid sequence comprising a suitable set of expression control elements) suitable for expression of a ω-transaminase of the invention in a cell. Examples of such vectors include derivatives of SV40, bacterial plasmids, phage DNA, baculovirus, yeast plasmids fungal replicons, vectors derived from combinations of plasmids and phage DNA, and viral nucleic acid (RNA or DNA) vectors. In one embodiment, the ω-transaminase of the invention sequence-encoding nucleic acid molecule is comprised in a naked DNA or RNA vector, including, for example, a linear expression element (as described in, for instance, Sykes and Johnston, Nat Biotech 12, 355-59 (1997)), a compacted nucleic acid vector (as described in for instance U.S. Pat. No. 6,077,835 and/or WO 00/70087), or a plasmid vector such as pBR322, pUC 19/18, or pUC 118/119. Such nucleic acid vectors and the usage thereof are well known in the art (see, for instance, U.S. Pat. No. 5,589,466 and U.S. Pat. No. 5,973,972). In one embodiment, the DNA comprises an expression control sequence.

In one embodiment, the vector is suitable for expression of an ω-transaminase of the invention of the invention in a bacterial cell. Examples of such vectors include expression vectors such as BlueScript (Stratagene), pIN vectors (Van Heeke & Schuster, 1989, J Biol Chem 264, 5503-5509), pET vectors (Novagen, Madison, Wis.) and the like. In one embodiment, the expression vector may also or alternatively be a vector suitable for expression in a yeast and other fungal system. Any vector suitable for expression in a yeast system may be employed. Suitable vectors include, for example, vectors comprising constitutive or inducible promoters such as yeast alpha factor, alcohol oxidase and PGH (reviewed in: F. Ausubel et al., ed., 1987, Current Protocols in Molecular Biology, Greene Publishing and Wiley InterScience New York; and Grant et al., 1987, Methods in Enzymol 153, 516-544). In other embodiments, the expression vector is suitable for expression in baculovirus-infected insect cells. (Kost, T; and Condreay, J P, 1999, Current Opinion in Biotechnology 10 (5): 428-33.)

Expression control sequences are engineered to control and drive the transcription of genes of interest, and subsequent expression of proteins in various cell systems. Plasmids combine an expressible gene of interest with expression control sequences (i.e. expression cassettes) that comprise desirable elements such as, for example, promoters, enhancers, selectable markers, operators, etc. In an expression vector of the invention, ω-transaminase of the invention-encoding nucleic acid molecules may comprise or be associated with any suitable promoter, enhancer, selectable marker, operator, repressor protein, polyA termination sequences and other expression-facilitating elements.

"Promoter" as used herein indicates a DNA sequence sufficient to direct transcription of a DNA sequence to which it is operably linked, i.e., linked in such a way as to permit transcription of the ω-transaminase of the invention -encoding nucleotide sequence when the appropriate signals are present. The expression of a ω-transaminase of the invention-encoding nucleotide sequence may be placed under control of any promoter or enhancer element known in the art. Examples of such elements include strong expression promoters (e.g., human CMV IE promoter/enhancer or CMV major IE (CMV-MIE) promoter, as well as RSV, SV40 late promoter, SL3-3, MMTV, ubiquitin (Ubi), ubiquitin C (UbC), and HIV LTR promoters). In some embodiments, the vector comprises a promoter selected from the group consisting of SV40, CMV, CMV-IE, CMV-MIE, RSV, SL3-3, MMTV, Ubi, UbC and HIV LTR.

Nucleic acid molecules of the invention may also be operably linked to an effective poly (A) termination sequence, an origin of replication for plasmid product in E. coli, an antibiotic resistance gene as selectable marker, and/or a convenient cloning site (e.g., a polylinker). Nucleic acids may also comprise a regulatable inducible promoter (inducible, repressable, developmentally regulated) as opposed to a constitutive promoter such as CMV IE (the skilled artisan will recognize that such terms are actually descriptors of a degree of gene expression under certain conditions).

Selectable markers are elements well-known in the art. Under the selective conditions, only cells that express the appropriate selectable marker can survive. Commonly, selectable marker genes express proteins, usually enzymes,that confer resistance to various antibiotics in cell culture. In other selective conditions, cells that express a fluorescent protein marker are made visible, and are thus selectable. Embodiments include beta-lactamase (bla) (beta-lactam antibiotic resistance or ampicillin resistance gene or ampR), bls (blasticidin resistance acetyl transferase gene), bsd (blasticidin-S deaminase resistance gene), bsr (blasticidin-S resistance gene), Sh ble (Zeocin® resistance gene), hygromycin phosphotransferase (hpt) (hygromycin resistance gene), tetM (tetracycline resistance gene or tetR), neomycin phosphotransferase II (npt) (neomycin resistance gene or neoR), kanR (kanamycin resistance gene), and pac (puromycin resistance gene).

In certain embodiments, the vector comprises one or more selectable marker genes selected from the group consisting of bla, bls, BSD, bsr, Sh ble, hpt, tetR, tetM, npt, kanR and pac. In other embodiments, the vector comprises one or more selectable marker genes encoding green fluorescent protein (GFP), enhanced green fluorescent protein (eGFP), cyano fluorescent protein (CFP), enhanced cyano fluorescent protein (eCFP), or yellow fluorescent protein (YFP).

For the purposes of this invention, gene expression in eukaryotic cells may be tightly regulated using a strong promoter that is controlled by an operator that is in turn regulated by a regulatory protein, which may be a recombinant "regulatory fusion protein" (RFP). The RFP consists essentially of a transcription blocking domain, and a ligand-binding domain that regulates its activity. Examples of such expression systems are described in US20090162901A1, which is herein incorporated by reference in its entirety.

As used herein "operator" indicates a DNA sequence that is introduced in or near a gene in such a way that the gene may be regulated by the binding of the RFP to the operator and, as a result, prevents or allow transcription of the gene of interest, i.e. a nucleotide encoding a polypeptide of the invention. A number of operators in prokaryotic cells and bacteriophage have been well characterized (Neidhardt, ed., Escherichia coli and Salmonella; Cellular and Molecular Biology 2d. Vol 2 ASM Press, Washington D.C. 1996). These include, but are not limited to, the operator region of the LexA gene of E. coli, which binds the LexA peptide, and the lactose and tryptophan operators, which bind the repressor proteins encoded by the Lad and trpR genes of E. coli. These also include the bacteriophage operators from the lambda PR and the phage P22 ant/mnt genes, which bind the repressor proteins encoded by lambda cl and P22 arc. In some embodiments, when the transcription blocking domain of the RFP is a restriction enzyme, such as Notl, the operator is the recognition sequence for that enzyme. One skilled in the art will recognize that the operator must be located adjacent to, or 3' to the promoter such that it is capable of controlling transcription by the promoter. For example, U.S. Pat. No. 5,972,650, which is incorporated by reference herein, specifies that tetO sequences be within a specific distance from the TATA box. In specific embodiments, the operator is preferably placed immediately downstream of the promoter. In other embodiments, the operator is placed within 10 base pairs of the promoter.

In an exemplary cell expression system, cells are engineered to express the tetracycline repressor protein (TetR) and a protein of interest is placed under transcriptional control of a promoter whose activity is regulated by TetR. Two tandem TetR operators (tetO) are placed immediately downstream of a CMV-MIE promoter/enhancer in the vector. Transcription of the gene encoding the protein of interest directed by the CMV-MIE promoter in such vector may be blocked by TetR in the absence of tetracycline or some other suitable inducer (e.g. doxycycline). In the presence of an inducer, TetR protein is incapable of binding tetO, hence transcription then translation (expression) of the protein of interest occurs. (See, e.g., U.S. Pat. No. 7,435,553, which is herein incorporated by reference in its entirety.)

The vectors of the invention may also employ Cre-lox recombination tools to facilitate the integration of a gene of interest into a host genome. A Cre-lox strategy requires at least two components: 1) Cre recombinase, an enzyme that catalyzes recombination between two IoxP sites; and 2) IoxP sites (e.g. a specific 34-base pair by sequence consisting of an 8-bp core sequence, where recombination takes place, and two flanking 13-bp inverted repeats) or mutant lox sites. (See, e.g. Araki et al., 1995, PNAS 92:160-4; Nagy, A. et al., 2000, Genesis 26:99-109; Araki et al., 2002, Nuc Acids Res 30(19):e103; and US20100291626A1, all of which are herein incorporated by reference). In another recombination strategy, yeast-derived FLP recombinase may be utilized with the consensus sequence FRT (see also, e.g. Dymecki, S. M., 1996, PNAS 93(12): 6191-6196).

As used herein, the term "host cell" includes any cell that is suitable for expressing a recombinant nucleic acid sequence. Cells include those of prokaryotes and eukaryotes (single-cell or multiple-cell), bacterial cells (e.g., strains of E. coli, Bacillus spp., Streptomyces spp., etc.), mycobacteria cells, fungal cells, yeast cells (e.g. S. cerevisiae, S. pombe, P. partoris, P. methanolica, etc.), plant cells, insect cells (e.g. SF-9, SF-21, baculovirus-infected insect cells, Trichoplusia ni, etc.), non-human animal cells, mammalian cells, human cells, or cell fusions such as, for example, hybridomas or quadromas. In certain embodiments, the cell is a human, monkey, ape, hamster, rat or mouse cell. In other embodiments, the cell is eukaryotic and is selected from the following cells: CHO (e.g. CHO K1, DXB-11 CHO, Veggie-CHO), COS (e.g. COS-7), retinal cells, Vero, CV1, kidney (e.g. HEK293, 293 EBNA, MSR 293, MDCK, HaK, BHK21), HeLa, HepG2, WI38, MRC 5, Colo25, HB 8065, HL-60, Jurkat, Daudi, A431 (epidermal), CV-1, U937, 3T3, L cell, C127 cell, SP2/0, NS-0, MMT cell, tumor cell, and a cell line derived from an aforementioned cell. In some embodiments, the cell comprises one or more viral genes, e.g. a retinal cell that expresses a viral gene (e.g. a PER.C6® cell). In some embodiments, the cell is a CHO cell. In other embodiments, the cell is a CHO K1 cell. In one embodiment, the host cell is a bacterium, especially a probiotic bacterium. In one embodiment, the bacterium is selected from Bifidobacterium breve. In one embodiment, the bacterium is Bifidobacterium breve UCC2003. In one embodiment, the bacterium is a probiotic bacterium, optionally selected from Streptococcus thermophilus, Bacillus laterosporus, Pediococcus acidilactici, Bifidobacterium infantis, Bifidobacterium bifidum, Bifidobacterium lactis, Bifidobacterium longum, Lactobacillus acidophilus, Lactobacillus brevis, Lactobacillus bulgaricus, Lactobacillus casei, Lactobacillus gasseri, Lactococcus lactis, Lactobacillus plantarum, Lactobacillus paracasei, Lactobacillus rhamnosus, Lactobacillus salivarius.

As used herein, the term "transformed cell of the invention" refers to a host cell comprising a nucleic acid stably integrated into the cellular genome that comprises a nucleotide sequence coding for expression of a Ω-transaminase of the invention of the invention. In another embodiment, the present invention provides a cell comprising a non-integrated (i.e., episomal) nucleic acid, such as a plasmid, cosmid, phagemid, or linear expression element, which comprises a sequence coding for expression of a ω-transaminase of the invention of the invention. In other embodiments, the present invention provides a cell line produced by stably transfecting a host cell with a plasmid comprising an expression vector of the invention.

As used herein, the term "engineered" as applied to a cell means genetically engineered using recombinant DNA technology, and generally involves the step of synthesis of a suitable expression vector (see above) and then transfecting the expression vector into a host cell (generally stable transfection). The cell may also be engineered using gene editing technology described in, for example, Gupta RM, Musunuru K. Expanding the genetic editing tool kit: ZFNs, TALENs, and CRISPR-Cas9. J Clin Invest. 2014 Oct;124(10):4154-61. doi: 10.1172/JCI72992. Epub 2014 Oct 1. Review. PubMed: 25271723. Free full-text available from PubMed Central: PMC4191047; Hsu PD, Lander ES, Zhang F. Development and applications of CRISPR-Cas9 for genome engineering. Cell. 2014 Jun 5;157(6):1262-78. doi:10.1016/j.cell.2014.05.010. Review. PubMed: 24906146. Free full-text available from PubMed Central: PMC4343198; Komor AC, Badran AH, Liu DR. CRISPR-Based Technologies for the Manipulation of Eukaryotic Genomes. Cell. 2017 Apr 20;169(3):559. doi:10.1016/j.cell.2017.04.005. PubMed: 28431253; and Lander ES. The Heroes of CRISPR. Cell. 2016 Jan 14;164(1-2):18-28. doi:10.1016/j.cell.2015.12.041. Review. PubMed: 26771483.

As used herein, the term "heterologous expression" refers to expression of a nucleic acid in a host cell that does not naturally have the specific nucleic acid. Insertion of the nucleic acid into the heterologous host is performed by recombinant DNA technology.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

The thermodynamically-favoured oxidative deamination reaction was investigated using the *cis-* (**8b**) and *trans-* (**8a**) diastereomers separately.

Racemic tetralone intermediate **6** was synthesised from 1-napthol and 1,2-dichlorobenzene in the presence of a strong Lewis acid. Subsequent reduction furnished a pair of diastereomeric alcohols, **7a** and **7b**. These diastereomers were separated *via* flash chromatography. Mitsunobu chemistry then provided the *trans-* and *cis*-azides with inversion of stereochemistry, which were converted to the target amines **8a** and **8b** *via* a Staudinger reduction (Scheme 1).

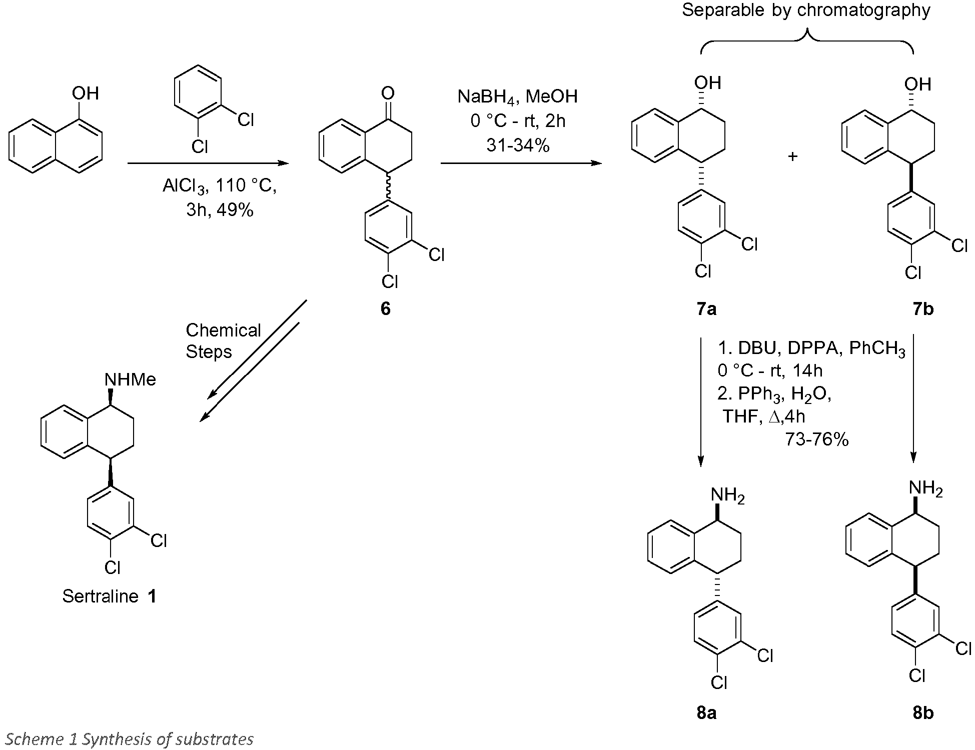

TA3 was then tested against the substrates **8a** and **8b** with the aim of finding some activity. 1 equivalent of pyruvate was used. Due to the high molecular weight of the substrate, solubility was expected to be an issue. 10% DMSO was used initially.

TA3 showed activity and enantioselectivity in initial experiments against the *cis*-substrate **8b.** Interestingly, TA3 showed activity against the cis-substrate only, giving the (4*S*)-tetralone product in 92% ee.. Excellent kinetic conversion was achieved in 16 h (Table 1). Contrastingly, the same reaction conditions with the trans-substrate **8a** resulted in <1% conversion (Table 2). The selectivity of the enzyme was confirmed by comparing the reaction HPLC trace with that of a sample of the (4*S*)-tetralone (see Supporting Information).

In order to further investigate the novelty of this transformation and to gauge if the activity was indeed unusual, we examined the viability of the well-studied transaminase from *Chromobacterium violaceum* (CV-TA) in the same reactions. This transaminase was carefully chosen as it is in the same fold class as TA3 and hence is also (*S*)-selective.^{32a} Moreover, its sequence shows 50% identity to that of TA3, although the key residues in the active site are retained. In these experiments, the same conditions were used. CV-TA was found to be active against both **8a** (Table 2) and **8b** (Table 1). Due to the (*S*)-selectivity at the reacting site the trans-stereoisomer **8a** gave rise to the (4R)-tetralone, thereby showing that this enzyme is not stereo-sensitive to the remote site.

**Table 1 Activity of TA3 and CV-TA against substrate 8b.**

| | | | | | |
|---|---|---|---|---|---|
| 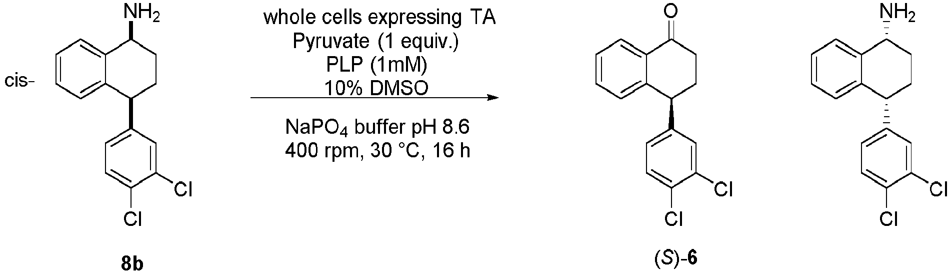 | | | | | |

| Transaminase | Conv. NMR (%) | Conv. Calc. (%) | *ee* product **6** (%) | ee substrate (%) | E |
|---|---|---|---|---|---|
| TA3 | 51 | 50 | 92 (4S) | 93 | 82 |
| CV-TA | 47 | 44 | 92 (4S) | 77 | 53 |

**Table 2 Activity of TA3 and CV-TA against substrate 8a.**

| | | | | | |
|---|---|---|---|---|---|
| 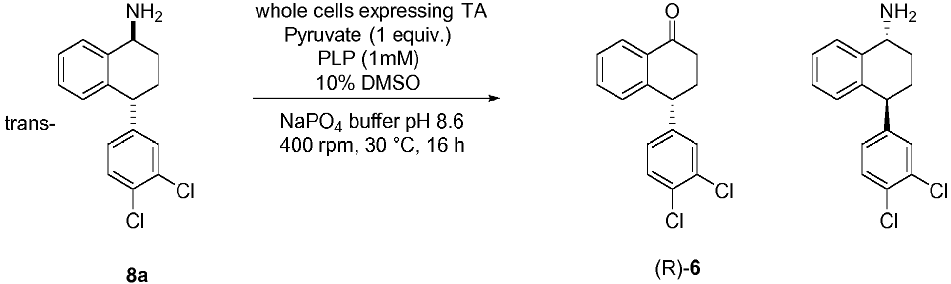 | | | | | |

| Transaminase | Conv. NMR (%) | Conv. Calc. (%) | *ee* product **6** (%) | *ee* substrate (%) | E |
|---|---|---|---|---|---|
| TA3 | <1 | - | - | 0 | - |
| CV-TA | 33 | 31 | 93 (4R) | 42 | 43 |

To confirm the remote selectivity of the new transaminase, a 50:50 mixture of the *cis*- and *trans-* amines was put into the reaction mixture (Table 3). In these reactions, TA3 showed the same selectivity as the separate reactions, giving the same product enantioselectivty (92% ee, 4*S*) with 23% NMR conversion (almost the kinetic limit of 25). The non-selectivity of TA from *chromobacterium violaceum* was similarly confirmed: 50% conversion with a tetralone enantioselectivity of 12% *ee*. We were unable to separate all four amine stereoisomers in the product mixture and therefore were unable to calculate the ee of the substrate(s).

**Table 3 Activity of TA3 and CV-TA against a mix of 8a and 8b.**

| | | | |
|---|---|---|---|
| 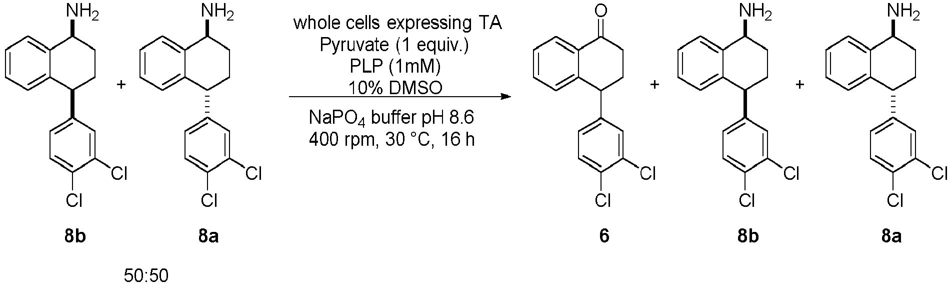 | | | |

| Transaminase | Conv. NMR (%) | *ee* product **6** (%) | NMR Ratio **6:8a:8b*** |
|---|---|---|---|
| TA3 | 23 | 92 (4S) | 1:2.5:1.3 |
| CV-TA | 44 | 12 (4S) | 1:0.68:0.63 |

| | | | |
|---|---|---|---|
| *Unable to calculate ee of substrates by chiral HPLC | | | |

Conveniently, TA3 is (S)-selective at the 4-position as well, meaning that the sertraline API can be accessed via two different methods. The reductive amination reaction could successfully resolve tetralone **6**, the key intermediate in the synthesis of sertraline. After separation of the product, oxidative deamination using the same biocatalyst will furnish the enantiopure tetralone. Reductive amination with methylamine furnishes the API.²⁹ Alternatively, it is possible to go directly from the (1*S*)-primary amine desmethylsertraline **2**.^{30b,30c}

In conclusion, we have demonstrated the first example of remote stereoselectivity using a transaminase biocatalyst. The unique selectivity of this enzyme enables the resolution of the tetralone **6**, a key intermediate in the synthesis of the important antidepressant, sertraline. This resolution could fit into a synthesis of the API.

### General Procedure for the Oxidative deamination of amines

*E. Coli* cells containing overexpressed transaminase (30 mgs) were suspended in 50 mM sodium phosphate buffer (pH 8.5) in a 15 mL centrifuge tube. The suspension was sonicated (30% intensity) using a probe for 10 s, followed by 30 s on ice. This process was repeated 5 times to lyophilise the cells. PLP solution (50 µL buffer, overall conc. 1mM) and sodium pyruvate (in 50 µL buffer, overall 1 equiv.) solutions were added, followed by the amine substrate (20 mM in 100 µL DMSO). The solution was shaken at 30 °C, 400 rpm for 16 h. The reaction was stopped through the addition of 5 M aqueous NaOH solution. Ethyl acetate (≈ 4 mL) was added and the tubes were centrifuged to pellet the cells. The organic phase was passed through a silica plug containing Na₂SO₄ and the solvent was removed *in vacuo.* The crude products were analysed by ¹H NMR and chiral HPLC.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

### References

1. Nugent, T.C. (ed) Chiral Amine Synthesis: Methods, Developments and Application 2010, WILEY-VCH Verlag GmbH & Co. KGaA, Weinheim.
2. a) Kellogg, R.M.; Leeman, M Crystallization as a Tool in Industrial Applications of Asymmetric Synthesis in Comprehensive Chirality, 2012, 9, 367-399; b)Kiwala, D.; Olbrycht, M.; Balawejder, M.; Piatkowski, W.; Seidel-Morgenstern, A.; Antos, D. Separation of stereoisomeric mixtures of nafronyl as a representative of compounds possessing two stereogenic centers by coupling crystallization, diastereoisomeric conversion and chromatography, Org. Proc. Res. Dev. 2016, 20(3), 615-625.
3. a) Sun, H.; Zhang, H.; Ang, E. L.; Zhao, H. Biocatalysis for the synthesis of pharmaceuticals and pharmaceutical intermediates, Bioorg. Med. Chem. 2017 26(7), 1275-1284 b) Torrelo, G.; Hanefeld, U.; Hollmann, F. Biocatalysis, Catal. Lett. 2015, 145, 309-345; c) Reetz, M. Biocatalysis in Organic Chemistry and Biotechnology: Past, Present, and Future, J. Am. Chem. Soc. 2013, 135(34), 12480-12496; d) Choi, J.-M.; Han, S.-S.; Kim, H.-S Industrial applications of enzyme biocatalysis: Current status and future aspects, Biotechnol. Adv. 2015, 33(7), 1443-1454.
4. a) Turner, N.J.; O'Reilly, E. Biocatalytic retrosynthesis, Nat Chem Biol. 2013, 9(5), 285-8; b) Green, A.P.; Turner, N.J. Biocatalytic retrosynthesis: Redesigning synthetic routes to high-value chemicals, Perspectives in Science 2016, 9, 42-48; c) de Souza, R.O.M.A.; Miranda, L.S.M.; Bornscheuer, U.T. A Retrosynthesis Approach for Biocatalysis in Organic Synthesis, Chem. Eur. J. 2017, 23, 12040-12063.
5. a) Ghislieri, D.; Turner, N.J. Biocatalytic approaches to the synthesis of enantiomerically pure chiral amines, Topics in Catal. 2014, 57(5), 284-300; b) Kroutil, W.; Fischereder, E.-M.; Fuchs, C.S.; Lechner, H.; Mutti, F.G.; Pressnitz, D.; Rajagopalan, A.; Sattler, J. H.; Simon, R.C.; Siirola, E. Asymmetric preparation of prim-, sec-, and tert-amines employing selected biocatalysts, Org. Proc. Res. Dev. 2013, 17(5), 751-759; c) Höhne, M.; Bornscheuer, U.T. Biocatalytic routes to optically active amines, ChemCatChem. 2009, 1, 42-51.
6. a) Blacker, A.J.; Stirling, M.J.; Page, M.I. Catalytic racemisation of chiral amines and application in dynamic kinetic resolution, Org. Proc. Res. Dev. 2007, 11(3), 642-648; b) Gotor-Fernandez, V.; Brieva, R.; Gotor, V. Lipases: Useful biocatalysts for the preparation of pharmaceuticals, J. Mol. Catal. B: Enz. 2006, 40(3-4), 111-120; c) van Rantwijk, F.; Sheldon, R.A. Enantioselective acylation of chiral amines catalysed by serine hydrolases, Tetrahedron 2004, 60(3), 501-519.
7. Thalién, L.K.; Zhao, D.; Sortais, J.-P.; Paetzold, J.; Hoben, C.; Bäckvall, J.-E. A chemoenzymatic approach to enantiomerically pure amines using dynamic kinetic resolution: application to the synthesis of norsertraline, Chem. Eur. J. 2009, 15, 3403-3410.
8. a) Herter, S.; Medina, F.; Wagschal, S.; Benhaim, C.; Leipold, F.; Turner, N.J. Mapping the substrate scope of monoamine oxidase (MAO-N) as a synthetic tool for the enantioselective synthesis of chiral amines, Bioorg. Med. Chem. 2018, 26(7), 1338-1346 b) Ghislieri, D.; Green, A.P.; Pontini, M.; Willies, S.C.; Rowles, I.; Frank, A.; Grogan, G.; Turner, N.J. Engineering an enantioselective amine oxidase for the synthesis of pharmaceutical building blocks and alkaloid natural products, J. Am. Chem. Soc. 2013, 135(29), 10863-10869.
9. Imin red a) Schrittwieser, J.H.; Velikogne, S.; Kroutil, W. Biocatalytic imine reduction and reductive amination of ketones, Adv. Synth. Catal. 2015, 357(8), 1655-1685; b)
10. Aleku, G.A.; France, S.P.; Man, H.; Mangas-Sanchez, J.; Montgomery, S.L.; Sharma, M.; Leipold, F.; Hussain, S.; Grogan, G.; Turner, N.J. A reductive aminase from Aspergillus oryzae, Nature Chem. 2017, 9(10), 961-969.
11. a) Slabu, I.; Galman, J.L.; Lloyd, R.C.; Turner, N.J. Discovery, Engineering and Synthetic Application of Transaminase Biocatalysts, ACS Catal., 7(12), 8263-8284 b) Guo, F.; Berglund, P. Transaminase biocatalysis: optimization and application, Green Chem. 2017, 19(2), 333-360; c) Mathew, S.; Yun, H. ω-Transaminases for the production of optically pure amines and unnatural amino acids, ACS Catal. 2012, 2, 993-1001; d) Simon, R.C.; Richter, N.; Busto, E.; Kroutil, W. Recent developments of cascade reactions involving ω-transaminases, ACS Catal. 2014, 4, 129-143; e) Paul, C.E.; Rodriguez-Mata, M.; Busto, E.; Lavandera, I.; Gotor-Fernandez, V.; Gotor, V.; Garcia-Cerrada, S.; Mendiola, J.; de Frutos, O.; Collado, I. Transaminases applied to the synthesis of high added-value enantiopure amines, Org. Proc. Res. Dev. 2014, 18(6), 788-792.
12. Hoehne, M.; Schaetzle, S.; Jochens, H.; Robins, K.; Bornscheuer, U.T. Rational assignment of key motifs for function guides in silico enzyme identification, Nature Chem. Biol. 2010, 6(11), 807-813.
13. a) Truppo, M.D.; Rozzell, J.D.; Turner, N.J. Efficient Production of Enantiomerically Pure Chiral Amines at Concentrations of 50 g/L Using Transaminases, Org. Proc. Res. Dev. 2010, 14, 234-237; b) Feng, Y.; Luo, Z.; Sun, G.; Chen, M.; Lai, J.; Lin, W.; Goldmann, S.; Zhang, L.; Wang, Z. Development of an Efficient and Scalable Biocatalytic Route to (3R)-3-Aminoazepane: A Pharmaceutically Important Intermediate, Org. Proc. Res. Dev. 2017, 21(4), 648-654.
14. Fuchs, M.; Farnberger, J.E.; Kroutil, W. Eur. The Industrial Age of Biocatalytic Transamination, J. Org. Chem. 2015, 32, 6965-6982.
15. Savile, C.K; Janey, J.M.; Mundorff, E.C.; Moore, J.C.; Tam, S.; Jarvis, W.R.; Colbeck, J.C.; Krebber A.; Fleitz, F.J.; Brands, J.; Devine, P.N.; Huisman, G.W.; Hughes, G.J. Biocatalytic asymmetric synthesis of chiral amines from ketones applied to sitagliptin manufacture, Science 2010, 329, 305-9.
16. Pavlidis, I.V.; Weiss, M.S.; Genz, M.; Spurr, P.; Hanlon, S.P.; Wirz, B.; Iding, H.; Bornscheuer, U.T. Identification of (S)-selective transaminases for the asymmetric synthesis of bulky chiral amines, Nature Chem. 2016, 8(11), 1076-1082
17. a) Torsvik, V.; ∅vreås, L.; Thingstad, T.F. Prokaryotic Diversity--Magnitude, Dynamics, and Controlling Factors, 2002, 296, 1064-1066; b) Handelsman, J.; Rondon, M.R.; Brady, S.F.; Clardy, J.; Goodman, R.M. Molecular biological access to the chemistry of unknown soil microbes: a new frontier for natural products, Chem. biol. 1998, 5(10), R245-249
18. a) Baud, D.; Jeffries, J.W.E.; Moody, T.S.; Ward, J.M.; Hailes, H.C. A metagenomics approach for new biocatalyst discovery: application to transaminases and the synthesis of allylic amines, Green Chem 2017, 19(4), 1134-1143; b) Jeffries, J.W.E.; Dawson, N.; Orengo, C.; Moody, T.S.; Quinn, D.J.; Hailes, H.C.; Ward, J.M. Metagenome Mining: A Sequence Directed Strategy for the Retrieval of Enzymes for Biocatalysis, ChemistrySelect 2016, 1(10), 2217-2220.
19. a) Parages, M.L.; Gutierrez-Barranquero, J.A.; Reen, F.J.; Dobson, A.D.W.; O'Gara, F. Integrated (meta) genomic and synthetic biology approaches to develop new biocatalysts, Marine Drugs 2016, 14(3), 62-91; b) Reen, F.J.; Gutierrez-Barranquero, J.A.; Dobson, A.D.W.; Adams, C.; O'Gara, F. Emerging concepts promising new horizons for marine biodiscovery and synthetic biology, Marine Drugs 2015, 13(5), 2924-2954.
20. a) Foley, A.M.; Gavin, D.P.; Joniec, I.; Maguire, A.R. Impact of variation of the acyl group on the efficiency and selectivity of the lipase-mediated resolution of 2-phenylalkanols, Tetrahedron: Asymm. 2017 28(9), 1144-1153; b) Deasy, R.E.; Moody, T.S.; Maguire, A.R. Influence of the position of the substituent on the efficiency of lipase-mediated resolutions of 3-aryl alkanoic acids, Tetrahedron: Asymm. 2013 24(23), 1480-1487; c) Deasy, R.E.; Brossat, M.; Moody, T.S.; Maguire, A.R. Lipase catalysed kinetic resolutions of 3-aryl alkanoic acids, Tetrahedron: Asymm. 2011 22(1), 47-61.
21. a) Mendez-Sanchez, D.; Lopez-Iglesias, M.; Gotor-Fernandez, V. Hydrolases in Organic Chemistry. Recent achievements in the synthesis of pharmaceuticals, Curr. Org. Chem. 2016, 20(11), 1186-1203; b) Cunha, R.L.O.R.; Ferreira, E.A.; Oliveira, C.S.; Omori, A.T. Biocatalysis for desymmetrization and resolution of stereocenters beyond the reactive center: How far is far enough? Biotechnol. Adv. 2015, 33(5), 614-623; c) Alfaro, B.M.; Gröger, H. Enzymatic resolution of racemates with a 'remote' stereogenic center as an efficient tool in drug, flavor and vitamin synthesis, Bioorg. Med. Chem. 2014, 22(20), 5539-5546 d) Barbayianni, E.; Kokotos, G. Biocatalyzed Regio- and Chemoselective Ester Cleavage: Synthesis of Bioactive Molecules, ChemCatChem 2012, 4(5), 592-608.
22. a) Gavin, D.P.; Foley, A.; Moody, T.S.; Rao Khandavilli, U.B.; Lawrence, S.E.; O'Neill, P.; Maguire, A.R. Hydrolase-mediated resolution of the hemiacetal in 2-chromanols: The impact of remote substitution, Tetrahedron: Asymm. 2017, 28(4), 577-585;
23. Richter, N.; Simon, R.C.; Lechner, H.; Kroutil, W.; Ward, J.M.; Hailes, H.C. ω-Transaminases for the amination of functionalised cyclic ketones, Org. Biomol. Chem. 2015, 13(33), 8843-8851
24. Limanto, J.; Ashley, E.R.; Yin, J.; Beutner, G.L.; Grau, B.T.; Kassim, A.M.; Kim, M.M.; Klapars, A.; Liu, Z.; Strotman, H.R.; Truppo, M.D. A Highly Efficient Asymmetric Synthesis of Vernakalant, Org. Lett. 2014, 16 (10), 2716-2719
25. Sertraline antidepressants
26. Koblan, K.S.; Hopkins, S.C.; Sarma, K.; Jin, F.; Goldman, R.; Kollins, S.H.; Loebel, A. Dasotraline for the Treatment of Attention-Deficit/Hyperactivity Disorder: A Randomized, Double-Blind, Placebo-Controlled, Proof-of-Concept Trial in Adults, Neuropsychopharmacy 2015, 40(12), 2745-2752.
27. Bogeso, K.P; Vibeke Christensen, A.; Hyttel, J.; Liljefors, T. 3-Phenyl-1-indanamines. Potential antidepressant activity and potent inhibition of dopamine, norepinephrine, and serotonin uptake, J. Med. Chem. 1985, 28(12), 1817-1828.
28. Lee, S.H; Kim, I.S.; Li, Q.R.; Dong, G.R.; Jeong, L.S.; Jung, Y.H. Stereoselective Amination of Chiral Benzylic Ethers Using Chlorosulfonyl Isocyanate: Total Synthesis of (+)-Sertraline, Journal of Organic Chemistry 2011, 76(24), 10011-10019.
29. Quallich, G.J. Development of the commercial process for Zoloft®/Sertraline, Chirality 2005, 17, 120-136.
30. a)Wang, G.; Zheng, C.; Zhao, G. Asymmetric reduction of substituted indanones and tetralones catalyzed by chiral dendrimer and its application to the synthesis of (+)-sertraline, Tetrahedron: Asymm. 2006, 17(14), 2074-2081; b) Chandrasekhar, S.; Venkat Reddy, M. An expedient total synthesis of cis-(+)-sertraline from D-phenylglycine Tetrahedron 2000, 56(8), 1111-1114; c) Fustero, S.; Lazaro, R.; Herrera, L.; Rodriguez, E.; Mateu, N.; Barrio, P. Asymmetric Allylation/Ring Closing Metathesis: One-Pot Synthesis of Benzo-fused Cyclic Homoallylic Amines. Application to the Formal Synthesis of Sertraline Derivatives, Org. Lett. 2013, 15(14), 3770-3773,
31. Blacker, A.J.; Brown, S.; Clique, B.; Gourlay, B.; Headley, C.E.; Ingham, S.; Ritson, D.; Screen, T.; Stirling, M.J.; Taylor, D.; Thompson, G. A Low-Waste Process To Sertraline By Diastereomeric Crystal Resolution and Waste Isomer Racemisation, Org. Proc. Res. Dev. 2009, 13, 1370-1378
32. a) Kaulmann, U.; Smithies, K.; Smith, M.E.B.; Hailes, H.C.; Ward, J.M. Substrate spectrum of ω-transaminase from Chromobacterium violaceum DSM30191 and its potential for biocatalysis Enz. Microb. Technol. 2007, 41(5), 628-637; b) Cho, B.-K.; Park, H.-Y.; Seo, J.-H.; Kim, J.; Kang, T.-J.; Lee, B.-S.; Kim, B.-G. Redesigning the Substrate Specificityof ω-Aminotransferase for the Kinetic Resolution of Aliphatic Chiral Amines, Biotechnol. Bioeng. 2008, 99(2), 275-284; c) D Koszelewski, D.; Tauber, K.; Faber, K.; Kroutil, W. ω-Transaminases for the synthesis of non-racemic α-chiral primary amines, Trends biotechnol. 2010, 28, 324-332

Organization Applicant
---------------------
      Street : Western Rd
      City : Cork
      State : Cork
      Country : Ireland
      PostalCode :
      PhoneNumber :
      FaxNumber :
      EmailAddress :
<110> OrganizationName : UNIVERSITY COLLEGE CORK - NATIONAL UNIVERSITY OF
IRELAND CORK

Application Project
-------------------
<120> Title : An omega-transaminase enzyme
<130> AppFileReference : P12496EP00
<140> CurrentAppNumber :
<141> CurrentFilingDate : ____-__-__

Sequence
--------
<213> OrganismName : Pseudovibrio
<400> PreSequenceString :
MDYIANLPPT HVLQEKDAAH HLHPFTDTKS LNAKGTRVIT RADGVYLWDS EGNKILDGMA      60
GLWCVNVGYG RQEIIDAVYR QMQQLPYYNT FFQSSHPPAI GLAERISSLA PDHLDHVFFA     120
GSGSEANDTV VRMVRHYWAS EGKPTKKTII SRHNAYHGST MAGASLGGMS AMHAQGGLPI     180
PDITHINQPY WYGEGGDMDP AAFGLMRARE LEAEIDRLGE DNVAAFIGEP IQGAGGVIIP     240
PETYWPEIQR ICRERNILLI ADEVICGFGR TGNWFGSQTF NFKPDLMPIA KGLSSGYLPI     300
GAVVVSEKVA KGFIEHGGEF YHGFTYSAHP AACAAALANL DIIENERLPE KVANDTGPYL     360
AEKWKTLGEH PLVGEARICG LVGALELSPD KARRARFEAE KGTVGTICRD HCFESGLVMR     420
HVGDSMIISP PLVISRSEVD ELIQKAHRAL DLTAADVTAQ SIK                       463
<212> Type : PRT
<211> Length : 463
      SequenceName : SEQ ID 1
      SequenceDescription :

Sequence
--------
<213> OrganismName : Pseudovibrio
<400> PreSequenceString :
atggactata tcgctaatct tccgcccact catgttctgc aagaaaagga tgccgcccac      60
catttgcatc cgtttacaga taccaagtcc ctcaacgcca aagggacgcg tgttattacg     120
cgggctgatg gtgtctatct ttgggattct gagggcaaca agattctgga tggtatggcc     180
gggctttggt gcgtgaacgt ggggtatggc cgtcaagaga ttatcgatgc ggtctaccgg     240
caaatgcaac aactgccgta ttacaatacg ttttttcaga gttctcaccc gcctgcaatt     300
ggtcttgctg aaaggatctc cagtctggca ccagatcatc tggaccatgt gttctttgca     360
ggctcgggct ctgaagccaa tgacaccgtg gtgcggatgg tgcgccatta ctgggctagt     420
gaaggtaagc cgaccaagaa gacaatcatt tcccgtcata atgcttatca cggctccact     480
atggctggtg cgagcttggg tggtatgagt gcgatgcatg cacagggcgg tctacctatt     540
cctgacatta cccatatcaa ccagccttat tggtatggtg aaggcggaga tatggaccct     600
gccgcgtttg gactgatgcg tgcacgtgag ctggaagctg agattgaccg gcttggagaa     660
gacaatgtag cggcctttat cggggagcct attcagggag cgggtggtgt gatcattccg     720
ccagaaacat attggcccga gattcagcgt atctgccgtg aacgcaatat tcttctgatt     780
gctgatgaag tgatttgcgg gtttgggcgt acgggtaact ggtttgggtc acagactttc     840
aacttcaaac cggatctgat gccgattgcc aaggggcttt cctcaggtta tctgccgatt     900
ggtgcggttg tggtgagtga gaaggttgcc aagggcttta ttgagcacgg cggtgaattt     960
tatcatggtt tcacttactc ggcacatccg gcagcttgtg ccgcagcact tgcgaacctt    1020
gatattattg agaatgagcg actgccggaa aaagtggcga atgatacagg gccgtatcta    1080
gctgagaaat ggaagactct tggcgagcat ccgttggttg gtgaggcgcg tatctgcggg    1140
ctggttggtg ctttggaact ttcgcctgat aaggcacgcc gtgctcgttt tgaagctgaa    1200
aaaggcactg ttggaacgat ctgccgcgat cattgctttg agagcggttt ggtgatgcgc    1260
catgttggcg acagcatgat catctcaccg ccacttgtga tctcgcgcag tgaggtggat    1320

gagttgattc agaaagcgca tcgtgcgttg gatctgacgg ctgctgatgt tactgcccaa 1380
agcatcaaat aa 1392
<212> Type : DNA
<211> Length : 1392
SequenceName : SEQ ID 2
SequenceDescription :

## Claims

1. An isolated ω-transaminase enzyme comprising (a) an amino acid sequence of SEQUENCE ID NO: 1 or (b) an amino acid sequence encoded by a nucleic acid sequence of SEQUENCE ID NO: 2.

2. A functional variant of the ω-transaminase enzyme of Claim 1 that is
(a) isolated;
(b) has at least 70% sequence identity with SEQUENC ID NO: 1; and
(c) is capable of enantioselectively resolving a racemic amine by transformation to a ketone by oxidative deamination, or enantioselectively resolving a racemic ketone by transformation to an amine by reductive amination, in which the amine and ketone typically contain a stereocentre removed from the reacting site.

3. A functional variant of Claim 2, capable of enantioselectively resolving a racemic ketone selected from a tetralone and an indanone by transformation to an amine by reductive amination, in which the ketone contains a stereocentre removed from the reacting site.

4. A functional variant of Claim 2 or 3, capable of enantioselectively transforming a (4S)-tetralone intermediate in the synthesis of sertraline, in which the functional variant displays remote stereospecificity for the tetralone intermediate.

5. A functional variant of any of Claims 2 to 5, having at least 90% sequence identity with SEQUENC ID NO: 1.

6. A functional variant of any of Claims 2 to 5 isolated from a *Pseudovibrio* bacterium or any other microorganism with the equivalent designated sequence.

7. An isolated ω-transaminase enzyme of any of Claims 1 to 6, in which the ω-transaminase is a recombinant or gene edited protein.

8. A nucleic acid encoding an isolated ω-transaminase of any of Claims 1 to 7.

9. An expression vector comprising DNA encoding an ω-transaminase of any of Claims 1 to 7, in which the vector is configured for heterologous expression of the ω-transaminase in a host cell.

10. A host cell engineered or edited to heterologously express an ω-transaminase of any of Claims 1 to 7.

11. A method of enantioselectively resolving a racemic amine by transformation to a ketone by oxidative deamination, or enantioselectively resolving a racemic ketone by transformation to an amine by reductive amination, in which the amine and ketone contain a stereocentre removed from the reacting site, the method comprising a step of reacting the racemic amine or ketone substrate with an isolated ω-transaminase enzyme of any of Claims 1 to 7.

12. A method of enantioselectively resolving a racemic amine by transformation to a ketone by oxidative deamination, in which the amine is selected from aminotetralin and an aminoindane comprising a stereocentre removed from the reacting site., the method comprising a step of reacting the racemic aminotetralin or aminoindane substrate with an isolated ω-transaminase enzyme of any of Claims 1 to 7.

13. A method of enantioselectively resolving a racemic ketone by transformation to an amine by reductive amination, in which the ketone is selected from a tetralone or an indanone comprising a stereocentre removed from the reacting site., the method comprising a step of reacting the racemic tetralone or indanone substrate with an isolated ω-transaminase enzyme of any of Claims 1 to 7.

14. A method according to Claim 13, in which the substrate is 4-(3,4-dichlorophenyl)-3,4-dihydro-1(2H)-naphthalenone (racemic tetralone), and the reaction selectively yields enantiopure (3,4-dichlorophenyl)-3,4-dihydro-1(2H)-naphthalamine (4R-tetralone).

15. A method of producing cis-(1S)(4S)-N-methyl-4-(3,4-dichlorophenyl)-1,2,3,4-tetrahydro-1-naphthaleneamine (sertraline) comprising a step of enantioselectively reducing racemic tetralone to selectively yield 4R-tetralone according to a method of Claim 14, and chemically converting 4R-tetralone to sertraline.
